# EUROPEAN PATENT SPECIFICATION

(11) **EP 2 190 820 B1**
(45) Date of publication and mention of the grant of the patent: **10.04.2013**
(21) Application number: 08797693.2
(22) Date of filing: 12.08.2008
(51) Int. Cl.: C07D 223/04, C07D 409/04, A61K 31/55, A61P 3/00

(54) **SUBSTITUTED AZEPINE-SULFONAMIDES USEFUL TO INHIBIT 11BETA-HYDROXYSTEROID DEHYDROGENASE TYPE-1**
ZUM INHIBIEREN VON 11BETA-HYDROXYSTEROIDDEHYDROGENASE VOM TYP 1 GEEIGNETE SUBSTITUIERTE AZEPINSULFONAMIDE
AZÉPINE-SULFONAMIDES UTILISÉS POUR INHIBER LA 11-BÊTA-HYDROXYSTÉROÏDE DÉSHYDROGÉNASE DE TYPE-1

(30) Priority: 15.08.2007 US 955974 P
(43) Date of publication of application: 02.06.2010
(73) Proprietor: Merck Sharp & Dohme Corp., Rahway, NJ 07065-0907 (US)
(72) Inventor: NEELAMKAVIL, Santhosh, Scotch Plains, NJ 07076 (US); BOYLE, Craig, D., Branchburg, NJ 08876 (US); CHACKALAMANNIL, Samuel, Califon, NJ 07830 (US)
(74) Representative: Jaap, David Robert
(86) International application number: PCT/US2008/072889
(87) International publication number: WO 2009/023664

(56) References cited:
- WO-A-2005/118538
- WO-A-2007/070506
- GRIGG R ET AL: "Sequential and Cascade Olefin Metathesis - Intramolecular Heck Reaction" TETRAHEDRON LETTERS, ELSEVIER, AMSTERDAM, vol. 39, no. 23, 4 June 1998 (1998-06-04), pages 4139-4142, XP004118832 ISSN: 0040-4039
- PAQUETTE L A ET AL: "Direct synthesis of previously inaccessible bridgehead azabicyclics by intramolecular cyclization of [alpha]-sulfonamido and [alpha]-sulfonimido radicals" JOURNAL OF ORGANIC CHEMISTRY 20010518 US, vol. 66, no. 10, 18 May 2001 (2001-05-18), pages 3564-3573, XP002499471 ISSN: 0022-3263
- GESSLER S ET AL: "Synthesis and metathesis reactions of a phosphine-free dihydroimidazole carbene ruthenium complex" TETRAHEDRON LETTERS, ELSEVIER, AMSTERDAM, vol. 41, no. 51, 16 December 2000 (2000-12-16), pages 9973-9976, XP004225200 ISSN: 0040-4039
- YAO Q ET AL: "Poly(fluoroalkyl acrylate)-Bound Ruthenium Carbene Complex: A Fluorous and Recyclable Catalyst for Ring-Closing Olefin Metathesis" JOURNAL OF THE AMERICAN CHEMICAL SOCIETY 20040114 US, vol. 126, no. 1, 14 January 2004 (2004-01-14), pages 74-75, XP002499472 ISSN: 0002-7863
- PAQUETTE L A ET AL: "Direct comparison of the response of bicyclic sultam and lactam dienes to photoexcitation. Concerning the propensity of differing bond types to bridgehead nitrogen for homolytic cleavage" JOURNAL OF ORGANIC CHEMISTRY 20061027 US, vol. 71, no. 22, 27 October 2006 (2006-10-27), pages 8438-8445, XP002499473 ISSN: 0022-3263
- PAQUETTE LEO A ET AL: "Synthesis of 1-Aza-8-thiabicyclo[4.2.1]nona-2,4-diene 8,8-dioxide and its conversion to a strained spirocycle via photoinduced SO2-N bond cleavage." ORGANIC LETTERS 15 APR 2004, vol. 6, no. 8, 15 April 2004 (2004-04-15), pages 1313-1315, XP002499474 ISSN: 1523-7060
- JOSEPH B. BINDER, ILIA A. GUZEI, RONALD T. RAINES: "Salicylaldimine Ruthenium Alkylidene Complexes: Metathesis Catalysts Tuned for Protic Solvents" ADVANCED SYNTHESIS & CATALYSIS, vol. 349, no. 3, 5 February 2007 (2007-02-05), pages 395-404, XP002499475
- DATABASE REGISTRY [Online] CHEMICAL ABSTRACTS SERVICE, COLUMBUS, OHIO, US; 13 June 2006 (2006-06-13), XP002499476 Database accession no. 887573-39-9
- DATABASE REGISTRY [Online] CHEMICAL ABSTRACTS SERVICE, COLUMBUS, OHIO, US; 13 June 2006 (2006-06-13), XP002499477 Database accession no. 887573-35-5

## Description

### Field of the Invention

The present invention relates to substituted azepine-sulfonamide compounds useful to inhibit 11β-hydroxysteroid dehydrogenase type-I, pharmaceutical compositions containing the compounds, and the compounds or pharmaceutical compositions for use in the treatment, prevention, inhibition, or amelioration of one or more conditions associated with the expression of 11β-hydroxysteroid dehydrogenase type-I.

### Background of the Invention

Glucocorticoids are steroid hormones that regulate many metabolic and homeostatic processes, including fat metabolism, function and distribution. Glucocorticoids also have profound and diverse physiological effects on development, neurobiology, inflammation, blood pressure, metabolism and programmed cell death.

Glucocorticoid action is dependent on the following factors: 1) circulating levels of glucocorticoid; 2) protein binding of glucocorticoids in circulation; 3) intracellular receptor density inside target tissues; and 4) tissue-specific pre-receptor metabolism by glucocorticoid-activating and glucocorticoid-inactivating enzymes collectively known as 11-beta-hydroxysteroid dehydrogenase (11-β-HSD). Two distinct isozymes of 11-β-HSD have been cloned and characterized. These two isozymes, known as 11-β-HSD type I and 11-β-HSD type II, respectively, catalyze the interconversion of active and inactive forms of various glucocorticoids. For example, in humans, the primary endogenously-produced glucocorticoid is cortisol. 11-β-HSD type I and 11-β-HSD type II catalyze the interconversion of hormonally active cortisol and inactive cortisone. 11-β-HSD type I is widely distributed in human tissues and its expression has been detected in lung, testis, central nervous system and most abundantly in liver and adipose tissue. Conversely, 11-β-HSD type II expression is found mainly in kidney, placenta, colon and salivary gland tissue.

Up-regulation of 11-β-HSD type I can lead to elevated cellular glucocorticoid levels and amplified glucocorticoid activity. This, in turn, can lead to increased hepatic glucose production, adipocyte differentiation and insulin resistance. In type II diabetes, insulin resistance is a significant pathogenic factor in the development of hyperglycemia. Persistent or uncontrolled hyperglycemia in both type 1 and type 2 diabetes has been associated with increased incidence of macrovascular and/or microvascular complications including atherosclerosis, coronary heart disease, peripheral vascular disease, stroke, nephropathy, neuropathy and retinopathy. Insulin resistance, even in the absence of profound hyperglycemia, is a component also of metabolic syndrome, which is characterized by elevated blood pressure, high fasting blood glucose levels, abdominal obesity, increased triglyceride levels and/or decreased HDL cholesterol. Further, glucocorticoids are known to inhibit the glucose-stimulated secretion of insulin from pancreatic beta-cells. Inhibition of 11-β-HSD type I is, therefore, expected to be beneficial in the treatment of metabolic syndromes, obesity, obesity-related disorders, hypertension, atherosclerosis, lipid disorders, type-II diabetes, insulin resistance, pancreatitis and associated conditions.

Mild cognitive impairment is a common feature of aging that may be ultimately related to the progression of dementia. Chronic exposure to glucocorticoid excess in certain brain subregions has been proposed to contribute to the decline of cognitive function. Inhibition of 11-β-HSD type I is expected to reduce exposure to glucocorticoids in the brain and protect against deleterious glucocorticoid effects on neuronal function, including cognitive impairment, dementia and/or depression, especially in connection with Alzheimer's Disease.

Glucocorticoids also have a role in corticosteroid-induced glaucoma. This particular pathology is characterized by a significant increase in intra-ocular pressure, which unresolved can lead to partial visual field loss and eventually blindness. Inhibition of 11-β-HSD type I is expected to reduce local glucocorticoid concentrations and, thus, intra-ocular pressure, producing beneficial effects in the management of glaucoma and other visual disorders.

Finally, glucocorticoids can have adverse effects on skeletal tissues. Continued exposure to excess glucocorticoids can produce osteoporosis and increased risk of fractures. Inhibition of 11-β-HSD type I should reduce local glucocorticoid concentration within osteoblasts and osteoclasts, producing beneficial effects for management of bone disease, including osteoporosis.

In view of the foregoing, there is a clear and continuing need for new compounds that target 11-β-HSD type I.

WO 2005/118538 discloses sulfonamide compounds useful to inhibit 11-β-HSD type I.

### Summary of the Invention

In its many embodiments, the present invention provides the claimed compounds as inhibitors of 11β-hydroxysteroid dehydrogenase type-I, pharmaceutical compositions containing the compounds, and said compounds or pharmaceutical compositions for use in the treatment, prevention, inhibition, or amelioration of one or more conditions associated with the expression of 11β-hydroxysteroid dehydrogenase type-I using such compounds or pharmaceutical compositions.

The claimed compounds and the salts, solvates, esters and prodrugs thereof are inhibitors of 11β-hydroxysteroid dehydrogenase type-I, and are for use in the treatment of metabolic syndromes, obesity, obesity-related disorders, hypertension, atherosclerosis, lipid disorders, type-II diabetes, insulin resistance, pancreatitis and associated conditions.

Table 1 shows structures of representative compounds of this invention.

**Table 1**

| **COMPOUND NO.** | **STRUCTURE** |
|---|---|
| **34** | 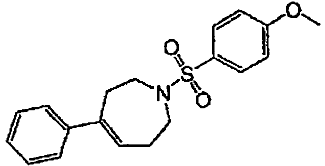 |
| **35** | 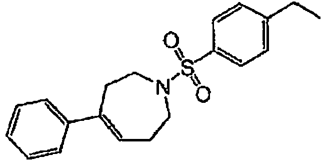 |
| **36** | 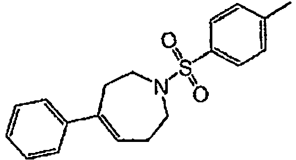 |
| **37** | 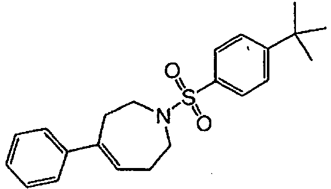 |
| **38** | 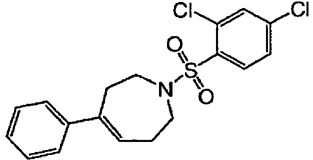 |
| **39** | 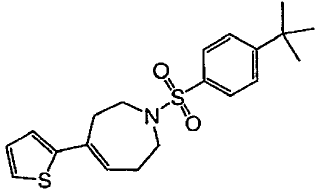 |
| **40** | 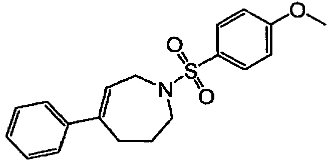 |
| **41** | 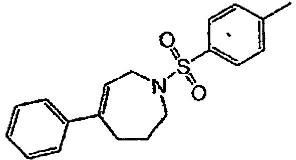 |
| **42** | 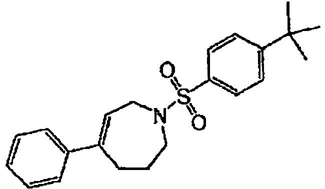 |

As used above, and throughout this disclosure, the following terms, unless otherwise indicated, shall be understood to have the following meanings:
"Patient" includes both human and animals.
"Mammal" means humans and other mammalian animals.

The term "purified", "in purified form" or "in isolated and purified form" for a compound refers to the physical state of said compound after being isolated from a synthetic process (e.g. from a reaction mixture), or natural source or combination thereof. Thus, the term "purified", "in purified form" or "in isolated and purified form" for a compound refers to the physical state of said compound after being obtained from a purification process or processes described herein or well known to the skilled artisan (e.g., chromatography, recrystallization and the like), in sufficient purity to be characterizable by standard analytical techniques described herein or well known to the skilled artisan.

It should also be noted that any carbon as well as heteroatom with unsatisfied valences in the text, schemes, examples and Tables herein is assumed to have the sufficient number of hydrogen atom(s) to satisfy the valences.

When a functional group in a compound is termed "protected", this means that the group is in modified form to preclude undesired side reactions at the protected site when the compound is subjected to a reaction. Suitable protecting groups will be recognized by those with ordinary skill in the art as well as by reference to standard textbooks such as, for example, T. W. Greene et al, Protective Groups in Organic Synthesis (1991), Wiley, New York.

As used herein, the term "composition" is intended to encompass a product comprising the specified ingredients in the specified amounts, as well as any product which results, directly or indirectly, from combination of the specified ingredients in the specified amounts.

Prodrugs and solvates of the compounds of the invention are also described herein. A discussion of prodrugs is provided in T. Higuchi and V. Stella, Pro-drugs as Novel Delivery Systems (1987) 14 of the A.C.S. Symposium Series, and in Bioreversible Carriers in Drug Design, (1987) Edward B. Roche, ed., American Pharmaceutical Association and Pergamon Press. The term "prodrug" means a compound (e.g, a drug precursor) that is transformed *in vivo* to yield a claimed compound or a pharmaceutically acceptable salt, hydrate or solvate of the compound. The transformation may occur by various mechanisms (e.g., by metabolic or chemical processes), such as, for example, through hydrolysis in blood. A discussion of the use of prodrugs is provided by T. Higuchi and W. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

One or more compounds of the invention may exist in unsolvated as well as solvated forms with pharmaceutically acceptable solvents such as water, ethanol, and the like, and it is intended that the invention embrace both solvated and unsolvated forms. "Solvate" means a physical association of a compound of this invention with one or more solvent molecules. This physical association involves varying degrees of ionic and covalent bonding, including hydrogen bonding. In certain instances the solvate will be capable isolation, for example when one or more solvent molecules are incorporated in the crystal lattice of the crystalline solid. "Solvate" encompasses both solution-phase and isolatable solvates. Non-limiting examples of suitable solvates include ethanolates, methanolates, and the like. "Hydrate" is a solvate wherein the solvent molecule is H₂O.

One or more compounds of the invention may optionally be converted to a solvate. Preparation of solvates is generally known. Thus, for example, M. Caira et al, J. Pharmaceutical Sci., 93(3), 601-611 (2004) describe the preparation of the solvates of the antifungal flucopazole in ethyl acetate as well as from water. Similar preparations of solvates, hemisolvate, hydrates and the like are described by E. C. van Tonder et al, AAPS PharmSciTech., 5(1), article 12 (2004); and A. L. Bingham et al, Chem. Commun., 603-604 (2001). A typical, non-limiting, process involves dissolving the inventive compound in desired amounts of the desired solvent (organic or water or mixtures thereof) at a higher than ambient temperature, and cooling the solution at a rate sufficient to form crystals which are then isolated by standard methods. Analytical techniques such as, for example I. R. spectroscopy, show the presence of the solvent (or water) in the crystals as a solvate (or hydrate).

"Effective amount" or "therapeutically effective amount" is meant to describe an amount of compound or a composition of the present invention effective in inhibiting the above-noted diseases and thus producing the desired therapeutic, ameliorative, inhibitory or preventative effect.

The claimed compounds can form salts which are also within the scope of this invention. Reference to a compound herein is understood to include reference to salts thereof, unless otherwise indicated. The term "salt(s)", as employed herein, denotes acidic salts formed with inorganic and/or organic acids, as well as basis salts formed with inorganic and/or organic bases. In addition, when a compound contains both a basic moiety, such as, but not limited to a pyridine or imidazole, and an acidic moiety, such as, but not limited to a carboxylic acid, zwitterions ("inner salts") may be formed and are included within the term "salts(s)" as used herein. Pharmaceutically acceptable (i.e., non-toxic, physiologically acceptable) salts are preferred, although other salts are also useful. Salts of the compounds may be formed, for example, by reacting a compound with an amount of acid or base, such as an equivalent amount, in a medium such as one in which the salt precipitates or in an aqueous medium followed by lyophilizalion.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates, naphthatenesulfonates, nitrates, oxalates, phosphates, propionates, salicylates, succinates, sulfates, tartarates, thiocyanates, toluenesulfonates (also known as tosylates), and the like. Additionally, acids which are generally considered suitable for the formation of pharmaceutically useful salts from basic pharmaceutical compounds are discussed, for example, by P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1-19: P. Gould, International J. of Pharmaceutics (1986) 33 201-217; Anderson et al, The Practice of Medicinal Chemistry (1996), Academic Press, New York; and in The Orange Book (Food & Drug Administration, Washington, D.C. on their website). These disclosures are incorporated herein by reference thereto.

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamines, t-butyl amines, and salts with amino acids such as arginine, lysine and the like. Basic nitrogen-containing groups may be quarternized with agents such as lower alkyl halides (e.g. methyl, ethyl, and butyl chlorides, bromides and iodides), dialkyl sulfates (e.g. dimethyl, diethyl, and dibutyl sulfates), long chain halides (e.g. decyl, lauryl, and stearyl chlorides, bromides and iodides), aralkyl halides (e.g. benzyl and phenethyl bromides), and others.

All such acid salts and base salts are intended to be pharmaceutically acceptable salts within the scope of the invention and all acid and base salts are considered equivalent to the free forms of the corresponding compounds for purposes of the invention.

The claimed compounds, and salts and solvates thereof, may exist in their tautomeric form (for example, as an amide or imino ether). All such tautomeric forms are contemplated herein as part of the present invention.

The claimed compounds may contain asymmetric or chiral centers, and, therefore, exist in different stereoisomeric forms. It is intended that all stereoisomeric forms of the claimed as well as mixtures thereof, including racemic mixtures, form part of the present invention. In addition, the present invention embraces all geometric and positional isomers. For example, if a claimed compound incorporates a double bond or a fused ring, both the cis- and trans-forms, as well as mixture, are embraced within the scope of the invention.

Diastereomeric mixtures can be separated into their individual diastereomers on the basis of their physical chemical differences by methods well known to those skilled in the art, such as, for example, by chromatography and/or fractional crystallization. Enantiomers can be separated by converting the enantiomeric mixture into a diastereomeric mixture by reaction with an appropriate optically active compound (e.g., chiral auxiliary such as a chiral alcohol or Mosher's acid chloride), separating the diastereomers and converting (e.g., hydrolyzing) the individual diastereomers to the corresponding pure enantiomers. Also, some of the claimed compounds may be atropisomers (e.g., substituted biaryls) and are considered as part of this invention. Enantiomers can also be separated by use of chiral HPLC column.

It is also possible that the claimed compounds may exist in different tautomeric forms, and all such forms are embraced within the scope of the invention. Also, for example, all keto-enol and imine-enamine forms of the compounds are included in the invention.

All stereoisomers (for example, geometric isomers, optical isomers and the like) of the present compounds (including those of the salts and, solvates of the compounds), such as those which may exist due to asymmetric carbons on various substituents, including enantiomeric forms (which may exist even in the absence of asymmetric carbons), rotameric forms, atropisomers, and diastereomeric forms, are contemplated within the scope of this invention, as are positional isomers (such as, for example, 4-pyridyl and 3-pyridyl). (For example, if a claimed compound incorporates a double bond or a fused ring, both the cis and trans-forms, as well as mixtures, are embraced within the scope of the invention. Also, for example, all keto-enol and imine-enamine form of the compounds are included in the invention. Individual stereoisomers of the compounds of the invention may, for example, be substantially free of other isomers, or may be admixed, for example, as racemates or with all other, or other selected, stereisomers. The chiral centers of the present invention can have the S or R configuration as defined by the *IUPAC* 1974 Recommendations. The use of the terms "salt", "solvate", "ester", "prodrug" and the like, is intended to equally apply to the salt, solvate, ester and prodrug of enantiomers, stereoisomers, rotamers, tautomers, positional isomers, racemates or prodrugs of the inventive compounds.

The present invention also embraces isotopically-labelled compounds of the present invention which are identical to those recited herein, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorus, fluorine and chlorine, such as ²H, ³H, ¹³C, ¹⁴C, ¹⁵N, ¹⁸O, ³¹P, ³²P, ³⁵S, ¹⁸F, and ³⁶Cl, respectively.

Certain isotopically-labelled compounds of Formula (I) (e.g., those labeled with ³H and ¹⁴C) are useful in compound and/or substrate distribution assays. Tritiated (i.e., ³H) and carbon-14 (i.e., ¹⁴C) isotopes are particularly preferred for their ease of preparation and detectability. Further, substitution with heavier isotopes such as deuterium (i.e. ²H) may afford certain therapeutic advantages resulting from greater metabolic stability (e.g., increased in vivo half-life or reduced dosage requirements) and hence may be preferred in some circumstances, isotopically labelled compounds can generally be prepared by following procedures analogous to those disclosed in the Schemes and/or in the Examples hereinbelow, by substituting an appropriate isotopically labelled reagent for a non-isotopically labelled reagent.

Polymorphic forms of the claimed compounds, and of the salts, solvates, esters and prodrugs of the compounds, are intended to be included in the present invention.

The compounds according to the invention have pharmacological properties; in particular, the claimed compounds can be inhibitors of 11β-hydroxysteroid dehydrogenase type I.

The term "obesity" as used herein, refers to a patient being overweight and having a body mass index (BMI) of 25 or greater. In one embodiment, an obese patient has a BMI of 25 or greater. In another embodiment, an obese patient has a BMI from 25 to 30. In another embodiment, an obese patient has a BMI greater than 30. In still another embodiment, an obese patient has a BMI greater than 40.

The term "obesity-related disorder" as used herein refers to: (i) disorders which result from a patient having a BMI of 25 or greater; and (ii) eating disorders and other disorders associated with excessive food intake. Non-limiting examples of an obesity-related disorder include edema, shortness of breath, sleep apnea, skin disorders and high blood pressure.

The term "metabolic syndrome" as used herein, refers to a set of risk factors that make a patient more succeptible to cardiovascular disease and/or type 2 diabetes. A patient is said to have metabolic syndrome if the patient has one or more of the following five risk factors:
1) central/abdominal obesity as measured by a waist circumference of greater than 40 inches in a male and greater than 35 inches in a female;
2) a fasting triglyceride level of greater than or equal to 150 mg/dL;
3) an HDL cholesterol level in a male of less than 40 mg/dL or in a female of less than 50 mg/dL;
4) blood pressure greater than or equal to 130/85 mm Hg; and
5) a fasting glucose level of greater than or equal to 110 mg/dL.

A preferred dosage is about 0.001 to 5 mg/kg of body weight/day of the claimed compound. An especially preferred dosage is about 0.01 to 5 mg/kg of body weight/day of a compound, or a pharmaceutically acceptable salt, solvate, ester or prodrug of said compound.

In one embodiment, the present invention provides one or more compounds, or a pharmaceutically acceptable salt or solvate thereof and at least one additional therapeutic agent that is not a compound for use in treading or preventing condition.

Non-limiting examples of additional therapeutic agents useful in the present uses for treating or preventing a Condition include, anti-obesity agents, antidiabetic agents, any agent useful for treating metabolic syndrome, any agent useful for treating a cardiovascular disease, cholesterol biosynthesis inhibitors, cholesterol absorption inhibitors, bile acid sequestrants, probucol derivatives, IBAT inhibitors, nicotinic acid receptor (NAR) agonists, ACAT inhibitors, cholesteryl ester transfer proten (CETP) inhibitors, low-denisity lipoprotein (LDL) activators, fish oil, water-soluble fibers, plant sterols, plant stanols, fatty acid esters of plant stanols, or any combination of two or more of these additional therapeutic agents.

Non-limiting examples of anti-obesity agents useful in the present uses for treating a Condition include CB1 antagonists or inverse agonists such as rimonabant, neuropeptide Y antagonists, MCR4 agonists, MCH receptor antagonists, histamine H₃ receptor antagonists or inverse agonists, metabolic rate enhancers, nutrient absorption inhibitors, leptin, appetite suppressants and lipase inhibitors.

Non-limting examples of appetite suppressant agents useful in the present uses for treating or preventing a Condition include cannabinoid receptor 1 (CB₁) antagonists or inverse agonists (e.g., rimonabant); Neuropeptide Y (NPY1, NPY2, NPY4 and NPY5) antagonists; metabotropic glutamate subtype 5 receptor (mGluR5) antagonist (*e.g*., 2-methyl-6-(phenylethynyl)-pyridine and 3[(2-methyl-1,4-thiazol-4-yl)ethynyl]pyridine); melanin-concentrating hormone receptor (MCH1R and MCH2R) antagonists; melanocortin receptor agonists (*e.g*., Melanotan-II and Mc4r agonists); serotonin uptake inhibitors (*e.g*., dexfenfluramine and fluoxetine); serotonin (5HT) transport inhibitors (*e.g*., paroxetine, fluoxetine, fenfluramine, fluvoxamine, sertaline and imipramine); norepinephrine (NE) transporter inhibitors (*e.g*., desipramine, talsupram and nomifensine); ghrelin antagonists; leptin or derivatives thereof; opioid antagonists (*e.g*., nalmefene, 3-methoxynaltrexone, naloxone and nalterxone); orexin antagonists; bombesin receptor subtype 3 (BRS3) agonists; Cholecystokinin-A (CCK-A) agonists; ciliary neurotrophic factor (CNTF) or derivatives thereof (*e.g*., butabindide and axokine); monoamine reuptake inhibitors (*e.g*., sibutramine); glucagon-like peptide 1 (GLP-1) agonists; topiramate; and phytopharm compound 57.

Non-limiting examples of metabolic rate enhancers useful in the present uses for treating or preventing a Condition include acetyl-CoA carboxylase-2 (ACC2) inhibitors; beta adrenergic receptor 3 (β3) agonists; diacylglycerol acyltransferase inhibitors (DGAT1 and DGAT2); fatty acid synthase (FAS) inhibitors (*e.g*., Cerulenin); phosphodiesterase (PDE) inhibitors (*e.g*., theophylline, pentoxifylline, zaprinast, sildenafil, amrinone, milrinone, cilostamide, rolipram and cilomilast); thyroid hormone β agonists; uncoupling protein activators (UCP-1,2 or 3) (*e.g*., phytanic acid, 4-[(E)-2-(5,6,7,8-tetramethyl-2-naphthalenyl)-1-propenyl]benzoic acid and retinoic acid); acyl estrogens (*e.g*., oleoyl-estrone); glucocorticoid antagonists; 11-beta hydroxy steroid dehydrogenase type 1 (11β HSD-1) inhibitors; melanocortin-3 receptor (Mc3r) agonists; and stearoyl-CoA desaturase-1 (SCD-1) compounds.

Non-limiting examples of nutrient absorption inhibitors useful in the present uses for treating or preventing a Condition include lipase inhibitors (*e.g*., orlistat, lipstatin, tetrahydrolipstatin, teasaponin and diethylumbelliferyl phosphate); fatty acid transporter inhibitors; dicarboxylate transporter inhibitors; glucose transporter inhibitors; and phosphate transporter inhibitors.

Non-limiting examples of cholesterol biosynthesis inhibitors useful in the present uses for treating or preventing a Condition include HMG-CoA reductase inhibitors, squalene synthase inhibitors, squalene epoxidase inhibitors and mixtures thereof.

Non-limiting examples of cholesterol absorption inhibitors useful in the present uses for treating or preventing a Condition include ezetimibe and other compounds suitable for the same purpose. In one embodiment, the cholesterol absorption inhibitor is ezetimibe.

HMG-CoA reductase inhibitors useful in the present uses for treating or preventing a Condition include, but are not limited to, statins such as lovastatin, pravastatin, fluvastatin, simvastatin, atorvastatin, cerivastatin, Cl-981, resuvastatin, rivastatin, pitavastatin, rosuvastatin, or L-659,699 ((E,E)-11-[3'R-(hydroxy-methyl)-4'-oxo-2'R-oxetanyl]-3,5,7R-trimethyl-2,4-undecadienoic acid).

Squalene synthesis inhibitors useful in the present uses for treating or preventing a Condition include, but are not limited to, squalene synthetase inhibitors; squalestatin 1; and squalene epoxidase inhibitors, such as NB-598 ((E)-N-ethyl-N-(6,6-dimethyl-2-hepten-4-ynyl)-3-[(3,3'-bithiophen-5-yl)methoxy]benzene-methanamine hydrochloride).

Bile acid sequestrants useful in the present uses for treating or preventing a Condition include, but are not limited to, cholestyramine (a styrenedivinylbenzene copolymer containing quaternary ammonium cationic groups capable of binding bile acids, such as QUESTRAN® or QUESTRAN LIGHT® cholestyramine which are available from Bristol-Myers Squibb), colestipol (a copolymer of diethylenetriamine and 1-chloro-2,3-epoxypropane, such as COLESTID® tablets which are available from Pharmacia), colesevelam hydrochloride (such as WelChol® Tablets (poly(allylamine hydrochloride) crosslinked with epichlorohydrin and alkylated with 1-bromodecane and (6-bromohexyl)-trimethylammonium bromide) which are available from Sankyo), water soluble derivatives such as 3,3-ioene, N-(cycloalkyl) alkylamines and poliglusam, insoluble quaternized polystyrenes, saponins and mixtures thereof. Suitable inorganic cholesterol sequestrants include bismuth salicylate plus montmorillonite clay, aluminum hydroxide and calcium carbonate antacids.

Probucol derivatives useful in the present uses for treating or preventing a Condition include, but are not limited to, AGI-1067 and others disclosed in U.S. patents Nos. 6,121,319 and 6,147,250.

IBAT ihibitors useful in the present uses for treating or preventing a Condition include, but are not limited to, benzothiepines such as therapeutic compounds comprising a 2,3,4,5-tetrahydro-1-benzothiepine 1,1-dioxide structure such as are disclosed in international Publication No. WO 00/38727.

Nicotinic acid receptor agonists useful in the present uses for treating or preventing a Condition include, but are not limited to, those having a pyridine-3-carboxylate structure or a pyrazine-2-carboxylate structure, including acid forms, salts, esters, zwitterions and tautomers, where available. Other examples of nicotinic acid receptor agonists useful in the present uses include nicotinic acid, niceritrol, nicofuranose and acipimox. An examples of a suitable nicotinic acid product is NIASPAN® (niacin extended-release tablets) which are available from Kos Pharmaceuticals Inc. (Cranbury, NJ).

ACAT inhibitors useful in the present the present uses for treating or preventing a Condition include, but are not limited to, avasimibe, HL-004, lecimibide and CL-277082 (*N*-(2,4-difluorophenyl)-*N*-[[4-(2,2-dimethylpcopyl)phenyl]-methyl]-*N-*heptylurea). See P. Chang et al., "Current, New and Future Treatments in Dyslipidaemia and Atherosclerosis", Drugs 2000 Jul;60(1); 55-93.

CETP inhibitors useful in the present uses for treating or preventing a Condition include, but are not, limited to, those disclosed in International Publication No. WO 00/38721 and U.S. Patent No. 6, 147,090.

LDL-receptor activators useful in the present uses for treating or preventing a Condition include, but are not limited to, include HOE-402, an imidazolidinyl-pyrimidine derivative that directly stimulates LDL receptor activity. See M. Huettinger et al., "Hypolipidemic activity of HOE-402 is Mediated by Stimulation of the LDL Receptor Pathway", Arteriosclar.Thromb. 1993; 13:1005-12.

Natural water-soluble fibers useful in the present uses for treating or preventing a Condition include, but are not limited to, psyllium, guar, oat and pectin.

Fatty acid esters of plant stanols useful in the present uses for treating or preventing a Condition include, but are not limited to, the sitostanol ester used in BENECOL® margarine.

Non-limiting examples of antidiabetic agents useful in the present uses for treating a Condition include insulin sensitizers, β-glucosidase inhibitors, DPP-IV inhibitors, insulin secretagogues, hepatic glucose output lowering compounds, antihypertensive agents, sodium glucose uptake transporter 2 (SGLT-2) inhibitors, insulin and insulin-containing compositions, and anti-obesity agents as set forth above.

In one embodiment, the antidiabetic agent is an insulin secretagogue. In one embodiment, the Insulin secretagogue is a sulfonylurea.

Non-limiting examples of sulfonylureas useful in the present uses include glipizide, tolbutamide, glyburide, glimepiride, chlorpropamide, acetohexamide, gliamilide, gliclazide, gliquidone, glibenclamide and tolazamide.

In another embodiment, the insulin secretagogue is a meglitinide.

Non-limiting examples of meglitinides useful in the present uses for treating a Condition include repaglinide, mitiglinide, and nateglinide.

In still another embodiment, the insulin secretagogue is GLP-1 or a GLP-1 mimetic.

Non-limiting examples of GLP-1 mimetics useful in the present methods include Byetta-Exanatide, Liraglutinide, CJC-1131 (ConjuChem, Exanatide-LAR (Amylin), BIM-51077 (Ipsen/LaRoche), ZP-10 (Zealand Pharmaceuticals), and compounds disclosed in International Publication No. WO 00/07617.

Other non-limiting examples of insulin secretagogues useful in the present uses include exendin, GIP and secretin.

In another embodiment, the antidiabetic agent is an Insulin sensitizer.

Non-limiting examples of insulin sensitizers useful in the present uses include PPAR activators or agonists, such as troglitazone, rosiglitazone, pioglitazone and englitazone; biguanidines such as metformin and phenformin; PTP-1 B inhibitors; and glucokinase activators.

In another embodiment, the antidiabetic agent is a β-Glucosidase inhibitor.

Non-limiting examples of β-Glucosidase inhibitors useful in the present uses include miglitol, acarbose, and voglibose.

In another embodiment, the antidiabetic agent is an hepatic glucose output lowering agent.

Non-limiting examples of hepatic glucose output lowering agents useful in the present uses include Glucophage and Glucophage XR.

In yet another embodiment, the antidiabetic agent is insulin, including all formulations of insulin, such as long acting and short acting forms of insulin.

Non-limiting examples of orally administrable insulin and insulin containing compositions include AL-401 from Autoimmune, and the compositions disclosed in U.S. Patent Nos. 4,579,730; 4,849,405; 4,963,526; 5,642,868; 5,763,396; 5,824,638; 5,843.866; 6,153,632; 6,191,105; and International Publication No. WO 85/05029.

In another embodiment, the antidiabetic agent is a DPP-IV inhibitor.

Non-limiting examples of DPP-IV inhibitors useful in the present uses include sitagliptin, saxagliptin, denagliptin, vildagliptin, alogliptin, alogliptin benzoate, Galvus (Novartis), ABT-279 and ABT-341 (Abbott), ALS-2-0426 (Alantos), ARI-2243 (Arisaph), BI-A and BI-B (Boehringer Ingelheim), SYR-322 (Takeda), MP-513 (Mitsubishi), DP-893 (Pfizer) and RO-0730699 (Roche).

In a further embodiment, the antidiabetic agent is a SGLT-2 inhibitor. Non-limiting examples of SGLT-2 inhibitors useful in the present uses include dapagliflozin and sergliflozin, AVE2268 (Sanofi-Aventis) and T-1095 (Tanabe Seiyaku).

Non-limiting examples of antihypertensive agents useful in the present uses for treating a Condition include β-blockers and calcium channel blockers (for example diltiazem, verapamil, nifedipine, amlopidine, and mybefradil), ACE inhibitors (for example captopril, lisinopril, enalapril, spirapril, ceranopril, zefenopril, fosinopril, cilazopril, and quinapril), AT-1 receptor antagonists (for example losartan, irbesartan, and valsartan), renin inhibitors and endothelin receptor antagonists (for example sitaxsentan).

In one embodiment, the antidiabetic agent is an agent that slows or blocks the breakdown of starches and certain sugars.

Non-limiting examples of antidiabetic agents that slow or block the breakdown of starches and certain sugars and are suitable for use in the compositions and methods of the present invention include alpha-glucosidase inhibitors and certain peptides for increasing insulin production. Alpha-glucosidase inhibitors help the body to lower blood sugar by delaying the digestion of ingested carbohydrates, thereby resulting in a smaller rise in blood glucose concentration following meals. Non-limiting examples of suitable alpha-glucosidase inhibitors include acarbose; miglitol; camiglibose; certain polyamines as disclosed in WO 01/47528 (incorporated herein by reference); voglibose. Non-limiting examples of suitable peptides for increasing insulin production including amlintide (CAS Reg. No. 122384-88-7 from Amylin; pramlintide, exendin, certain compounds having Glucagon-like peptide-1 (GLP-1) agonistic activity as disclosed in International Publication No. WO 00/07617.

Other specific additional therapeutic agents useful in the present uses for treating or preventing a Condition include, but are not limited to, rimonabant, 2-methyl-6-(phenylethynyl)-pyridine, 3[(2-methyl-1,4-thiazol-4-yl)ethynyl]pyridine, Melanotan-II, dexfenfluramine, fluoxetine, paroxetine, fenfluramine, fluvoxamine, sertaline, imipramine, desipramine, talsupram, nomifensine, leptin, nalmefene, 3-methoxynaltrexone, naloxone, nalterxone, butabindide, axokine, sibutramine, topiramate, phytopharm compound 57, Cerulenin, theophylline, pentoxifylline, zaprinast, sildenafil, amrinone, milrinone, cilostamide, rolipram, cilomilast, phytanic acid, 4-[(E)-2-(5,6,7,8-tetramethyl-2-naphthalenyl)-1-propenyl]benzoic acid, retinoic acid, oleoyl-estrone, orlistat, lipstatin, tetrahydrolipstatin, teasaponin and diethylumbelliferyl phosphate.

In one embodiment, the present combination therapies for use in treating or preventing diabetes comprise a claimed compound, an antidiabetic agent and/or an antiobesity agent.

In another embodiment, the present combination therapies for use in treating or preventing diabetes comprise a claimed compound, and an antidiabetic agent,

In another embodiment, the present combination therapies for use in treating or preventing diabetes comprise a claimed compound, and an anti-obesity agent.

In one embodiment, the present combination therapies for use in treating or preventing obesity comprise a claimed compound, an antidiabetic agent and/or an antiobesity agent.

In another embodiment, the present combination therapies for use in treating or preventing obesity comprise a claimed compound, and an antidiabetic agent.

In another embodiment, the present combination therapies for use in treating or preventing obesity comprise a claimed compound and an anti-obesity agent.

In one embodiment, the present combination therapies for use in treating or preventing metabolic syndrome comprise a claimed compound and one or more additional therapeutic agents selected from: anti-obesity agents, antidiabetic agents, any agent useful for treating metabolic syndrome, any agent useful for treating a cardiovascular disease, cholesterol biosynthesis inhibitors, sterol absorption inhibitors, bile acid sequestrants, probucol derivatives, IBAT inhibitors, nicotinic acid receptor (NAR) agonists, ACAT inhibitors, cholesteryl ester transfer proten (CETP) inhibitors, low-denisity lipoprotein (LDL) activators, fish oil, water-soluble fibers, plant sterols, plant stanols and fatty acid esters of plant stanols.

In one embodiment, the additional therapeutic agent is a cholesterol biosynthesis inhibitor. In another embodiment, the cholesterol biosynthesis inhibitor is an HMG-CoA reductase inhibitor. In another embodiment, the HMG-CoA reductase inhibitor is a statin. In another embodiment, the statin is lovastatin, pravastatin, simvastatin or atorvastatin.

In one embodiment, the additional therapeutic agent is a cholesterol absorption inhibitor. In another embodiment, the cholesterol absorption inhibitor is ezetimibe. In another embodiment, the cholesterol absorption inhibitor is a squalene synthetase inhibitor. In another embodiment, the cholesterol absorption inhibitor is a squalene epoxidase inhibitor.

In one embodiment, the additional therapeutic agent comprises a cholesterol absorption inhibitor and a cholesterol biosynthesis inhibitor. In another embodiment, the additional therapeutic agent comprises a cholesterol absorption inhibitor and a statin. In another embodiment, the additional therapeutic agent comprises ezetimibe and a statin. In another embodiment, the additional therapeutic agent comprises ezetimibe and simvastatin.

In one embodiment, the present combination therapies for use in treating or preventing metabolic syndrome comprise a claimed compound an antidiabetic agent and/or an antiobesity agent.

In another embodiment, the present combination therapies for use in treating or preventing metabolic syndrome comprise a claimed compound and an antidiabetic agent.

In another embodiment, the present combination therapies for use in treating or preventing metabolic syndrome comprise a claimed compound and an anti-obesity agent.

In one embodiment, the present combination therapies for use in treating or preventing a cardiovascular disease comprise one or more claimed compounds, and an additional agent useful for treating or preventing a cardiovascular disease.

When administering a combination therapy to a patient in need of such administration, the therapeutic agents in the combination, or a pharmaceutical composition or compositions comprising the therapeutic agents, may be administered in any order such as, for example, sequentially, concurrently, together, simultaneously and the like. The amounts of the various actives in such combination therapy may be different amounts (different dosage amounts) or same amounts (same dosage amounts).

In one embodiment, the one or more claimed compounds are administered during a time when the additional therapeutic agent(s) exert their prophylactic or therapeutic effect, or *vice versa.*

In another embodiment, the one or more claimed compounds and the additional therapeutic agent(s) are administered in doses commonly employed when such agents are used as monotherapy for treating a Condition.

In another embodiment, the one or more claimed compounds and the additional therapeutic agent(s) are administered in doses lower than the doses commonly employed when such agents are used as monotherapy for treating a Condition.

In still another embodiment, the one or more claimed compounds and the additional therapeutic agent(s) act synergistically and are administered in doses lower than the doses commonly employed when such agents are used as monotherapy for treating a Condition.

In one embodiment, the one or more claimed compounds and the additional therapeutic agent(s) are present in the same composition. In one embodiment, this composition is suitable for oral administration. In another embodiment, this composition is suitable for intravenous administration.

The one or more claimed compounds and the additional therapeutic agent(s) can act additively or synergistically. A synergistic combination may allow the use of lower dosages of one or more agents and/or less frequent administration of one or more agents of a combination therapy. A lower dosage or less frequent administration of one or more agents may lower toxicity of the therapy without reducing the efficacy of the therapy.

In one embodiment, the administration of one or more claimed compounds and the additional therapeutic agent(s) may inhibit the resistance of a Condition to these agents.

In one embodiment, when the patient is treated for diabetes or a diabetic complication, the additional therapeutic agent is an antidiabetic agent which is not a claimed compound. In another embodiment, the additional therapeutic agent is an agent useful for reducing any potential side effect of a claimed compound. Such potential side effects include, but are not limited to, nausea, vomiting, headache, fever, lethargy, muscle aches, diarrhea, general pain, and pain at an injection site.

The pharmacological properties of the compounds of this invention may be confirmed by a number of pharmacological assays. The exemplified pharmacological assays which are described later have been carried out with the compounds according to the invention and their salts.

The invention is also directed to pharmaceutical compositions which comprise at least one claimed compound, or a pharmaceutically acceptable salt, solvate, ester or prodrug of said compound and at least one pharmaceutically acceptable carrier.

The term "pharmaceutical composition" is also intended to encompass both the bulk composition and individual dosage units comprised of more than one (e.g., two) pharmaceutically active agents such as, for example, a compound of the present invention and an additional agent selected from the lists of the additional agents described herein, along with any pharmaceutically inactive excipients. The bulk composition and each individual dosage unit can contain fixed amounts of the afore-said "more than one pharmaceutically active agents". The bulk composition is maternal that has not yet been formed into individual dosage units. An illustrative dosage unit is an oral dosage unit such as tablets, pills and the like. Similarly, the herein-described method of treating a patient by administering a pharmaceutical composition of the present invention is also intended to encompass the administration of the afore-said bulk composition and individual dosage units.

For preparing pharmaceutical compositions from the compounds described by this invention, inert, pharmaceutically acceptable carriers can be either solid or liquid. Solid form preparations include powders, tablets, dispersible granules, capsules, cachets and suppositories. The powders and tablets may be comprised of from about 5 to about 95 percent active ingredient.
Suitable solid carriers are known in the art, e.g., magnesium carbonate, magnesium state, talc, sugar or lactose. Tablets, powders, cachets and capsules can be used as solid dosage forms suitable for oral administration. Examples of pharmaceutically acceptable carriers and methods of manufacture for various compositions may be found in A. Gennaro (ed.), Remington's Pharmaceutical Sciences, 18th Edition, (1990), Mack Publishing Co., Easton, Pennsylvania.

Liquid form preparations include solutions, suspensions and emulsions. As an example may be mentioned water or water-propylene glycol solutions for parenteral injection or addition of sweeteners and opacifiers for oral solutions, suspensions and emulsions. Liquid form preparations may also intranasal administration.

Aerosol preparations suitable for inhalation may include solutions and solids in powder form, which may be in combination with a pharmaceutically acceptable carrier, such as an inert compressed gas, e.g. nitrogen.

Also included are solid form preparations that are intended to be converted, shortly before use, to liquid form preparations for either oral or parenteral administration. Such liquid forms include solutions, suspensions and emulsions.

The compounds of the invention may also be deliverable transdermally. The transdermal compositions can take the form of creams, lotions, aerosols and/or emulsions and can be included in a transdermal patch of the matrix or reservoir type as are conventional in the art for this purpose.

The compounds of this invention may also be delivered subcutaneously.

Preferably the compound is administered orally.

Preferably, the pharmaceutical preparation is in a unit dosage form. In such form, the preparation is subdivided into suitable sized unit doses containing appropriate quantities of the active component, e.g. an effective amount to achieve the desired purpose.

The quantity of active compound in a unit dose of preparation may be varied or adjusted from about 1 mg to about 100 mg, preferably from about 1 mg to about 50 mg, more preferably from about 1 mg to about 25 mg, according to the particular application.

The actual dosage employed may be varied depending upon the requirements of the patient and the severity of the condition being treated. Determination of the proper dosage regimen for a particular situation is within the skill of the art. For convenience, the total daily dosage may be divided and administered in portions during the day as required.

The amount and frequency of administration of the compounds of the invention and/or the pharmaceutically acceptable salts thereof will be regulated according to the judgment of the attending clinician considering such factors as age, condition and size of the patient as well as severity of the symptoms being treated. A typical recommended daily dosage regimen for oral administration can range from about 1 mg/day to about 500 mg/day, preferably 1 mg/day to 200 mg/day, in two fo four divided doses.

Another aspect of this invention is a kit comprising a therapeutically effective amount of at least one claimed compound, or a pharmaceutically acceptable salt, solvate, ester or prodrug of said compound and a pharmaceutically acceptable carrier, vehicle or diluent.

Yet another aspect of this invention is a kit comprising an amount of at least one claimed compound, or a pharmaceutically acceptable salt, solvate, ester or prodrug of said compound and an amount of at least one therapeutic agent listed above, wherein the amounts of the two or more ingredients result in a desired therapeutic effect.

The invention disclosed herein is exemplified by the following preparations and:examples which should not be construed to limit the scope of the disclosure. Alternative mechanistic pathways and analogous structures will be apparent to those skilled in the art.

VVhere NMR data are presented; ¹H. spectra were obtained on either a Variant VXR-200 (200 MHz, ¹H), Varian Gemini-300 (300 MHZ), \/arian Mercury VX-400 (400MHz), or Bruker-Biospin AV-500(500MHz), and are reported as ppm with number of protons and multiplicities indicated parenthetically. Where LC/MS data are presented, analyses was performed using an Applied Biosystems API-100 mass spectrometer and C18 column, 10-95% CH₃CN-H₂O (with 0.05% TFA) gradient. The observed parent ion is given.

The following solvents and reagents may be referred to by their abbreviations in parenthesis:
Me = methyl
Et = ethyl
Pr = propyl
Bu = butyl
Ph = phenyl
Ac = acetyl
µl = microliters
AcOEt or EtOAc = ethyl acetate
AcOH or HOAc = acetic acid
ACN = acetonitrile
atm = atmosphere
Boc or BOC = tert-butoxycarbonyl
DCE = dichloroethane
DCM or CH₂Cl₂ = dichloromethane
DIPEA = diisopropylethylamine
DMAP = 4-dimethylaminopyridine
DMF = dimethylformamide
DMS = dimethylsulfide
DMSO = dimethyl sulfoxide
EDCI = 1-(3-dimethylaminopropyl)-3-ethylcarbodiimine
Fmoc or FMOC = 9-fluorenylmethoxycarbonyl
g = grams
h = hour
hal = halogen
HOBt = 1-hydroxybenzotriazole
LAH = lithium aluminum hydride
LCMS = liquid chromatography mass spectrometry
min = minute
mg = milligrams
mL = milliliters
mmol = millimoles
MCPBA = 3-chloroperoxybenzoic acid
MeOH = methanol
MS = mass spectrometry
NMR = nuclear magnetic resonance spectroscopy
RT or rt = room temperature (ambient, about 25°C)
TEA or Et₃N = triethylamine
TFA = trifluoroacetic acid
THF = tetrahydrofuran
TLC = thin layer chromatography
TMS = trimethylsilyl
Tr = triphenylmethyl

### Examples

The compounds of this invention can be prepared as generally described in the Preparation Schemes, and the following examples.

### General Methods

Solvents, reagents, and intermediates that are commercially available were used as received. Reagents and intermediates that are not commercially available were prepared in the manner described below. ¹H NMR spectra were obtained on a Gemini AS-400 (400 MHz) and are reported as ppm down field from Me4Si with number of protons, multiplicities, and coupling constants in Hertz indicated parenthetically. Where LC/MS data are presented, analyses was performed using an Applied Biosystems API-100 mass spectrometer and Shimadzu SCL-10A LC column: Altech platinum C18, 3 micron, 33 mm x 7mm ID; gradient flow: 0 min - 10% CH₃CN, 5 min - 95% CH₃CN, 7 min - 95% CH₃CN, 7.5 min -10% CH₃CN, 9 min - stop. The retention time and observed parent ion are given.

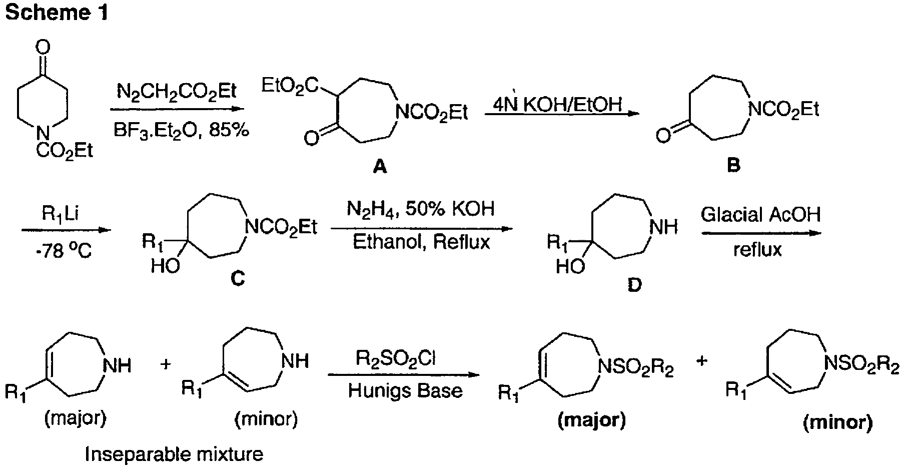

### Preparation of Compound 34 (Scheme 1)

### Method-A

To a solution of the *N*-carbethoxy-4-piperidone (3.0g, 17.3 mmol) in anh. ether (20 mL) maintained at -25 to -30 °C (dry ice-*i*-PrOH bath) was added simultaneously solutions of BF₃.Et₂O (3.23g, 22.8 mmol) and ethyl diazoacetate (3.0g, 26.3 mmol) in anh. ether (6.0 mL). After the additions were completed, the reaction was maintained at the same temperature for 1 hour and then was allowed to warm to room temperature. The reaction mixture was washed with 30% K₂CO₃ and extracted with EtOAc (3x100 mL). The organic phase was separated, dried on anh. Na₂SO₄, and concentrated *in vacuo* to give a crude orange oil which was then purified with 30% EtOAc / hexanes to give intermediate **A** as the product (3.7g, 82% yield).

### Method-B

To a solution of the intermediate **A** (1.0 g, 3.9 mmol) in ethanol (75.0 mL) was added 4M KOH (75.0 mL) and reaction mixture stirred at r.t. for 24 hours. The mixture was extracted with EtOAC and the organic phase was separated, dried (anh. Na₂SO₄) and concentrated *in vacuo* to give a residue. The residue was chromatographed on silica gel (40-60% EtOAc/hexanes) to give the intermediate **B** as a clear oil (0.4g, 56%).

### Method-C

A solution of the intermediate **B** (1.75g, 9.45 mmol) in anh. THF (20.0 mL) was cooled to -78 °C and 1.8 M PhLi (6.3 mL, 11.34 mmol) in *t*-butyl ether was added with stirring for 1 h and warmed to r.t. (1h), quenched with sat. NH₄Cl and extracted with CH₂Cl₂. Organic solvent evaporated under reduced pressure and preparative TLC purification provided the intermediate **C** in near quantitative yield.

### Method-D

To a solution of the intermediate **C** (0.06g, 0.23 mmol) was added hydrazine (1.0 mL), ethanol (2.0 mL) and 50% KOH (2.0 mL) and was refluxed over night, The mixture was allowed to cool to room temperature and extracted with CH₂Cl₂. The organic layer was then extracted with 3M HCl. The aqueous layer was basified and extracted with EtOAc. The organic layer was concentrated in vacuo to give the product amine **intermediate D** which was carried directly to the next step.

### Method-E

The amine intermediate **D** (0.3 g, 1.56 mmol) in acetic acid (3.0 mL) and conc. HCl (1.0 mL) was refluxed at 120 °C for 4.0h. The reaction was neutralized and basified with 1 N NaOH and extracted with CH₂Cl₂. The organics were washed with water and concentrated to give the product amines 4-phenyl-2,3,6,7-tetrahydro-1H-azepine (major) and 5-phenyl-2,3,4,7-tetrahydro-1H-azepine (minor) as inseparable mixture.

### Method-F

To the crude 4-phenyl-2,3,6,7-tetrahydro-1H-azepine and 5-phenyl-2,3,4,7-tetrahydro-1H-azepine (mixture of two amines, 0.0086g, 0.05 mmol) in CH₂Cl₂ (1.0 mL) was added Hunigs base (0.0194g, 0.15 mmol) and *p-*methoxy-phenyl sulfonylchloride (0.0155g, 0.075 mmol) and stirred for 2h. The reaction was then quenched with satd. NH₄Cl and extracted with CH₂Cl₂. The organics were concentrated in vacuo and purified by preparative TLC (15% EtOAc / hexanes) to give the major product **Compound 34** (0.005g, 30%) and minor product **Compound 40** (0.0025g, 15%).

**Compounds 35, 36, 37, 38, 39, 41** and **42** were prepared by the above method.

### Measurement of 11β-HSD1 activity

11β-HSD1 enzymatic activity was measured in a 50 µl reaction containing 20 mM NaPO₄ pH 7.5, 0.1 mM MgCl₂, 3mM NADPH (prepared fresh daily), 125 nM ³H-cortisone (American Radiochemicals) and 0.5 µg membrane. The reaction was incubated at room temperature for 1 hr before it was stopped by addition of 50 µM buffer containing 20 mM NaPO₄ pH 7.5, 30 µM 18β-glycyrrhetinic acid, 1 µg/ml monoclonal anti-cortisol antibody (Biosource) and 2 mg/ml anti-mouse antibody coated scintillation proximity assay (SPA) beads (Amersham Bioscience). The mixture was incubated at room temperature for 2 hrs with vigorous shaking and analyzed on TopCount scintillation counter.

Compounds according to the present invention showed activity against 11β-HSD1 in this assay.

Table 2 shows 11β-HSD-1 activity of representative compounds of this invention. The table and the compounds therein are not intended, nor should they be construed, to limit this invention in any manner whatsoever.

**Table 2**

| | **Human 11-β-HSD1** | **Mouse 11-β-HSD1** |
|---|---|---|
| **Compound No.** | **IC₅₀(nM)** | **IC₅₀(nM)** |
| **34** | 58 | 1876 |
| **37** | 68 | 905 |
| **36** | 76 | 1551 |
| **35** | 97 | 1242 |
| **38** | 994 | 1277 |
| **41** | 124 | 1511 |
| **40** | 178 | 1553 |
| **42** | 358 | 1648 |

## Claims

1. A compound selected from the group consisting of: 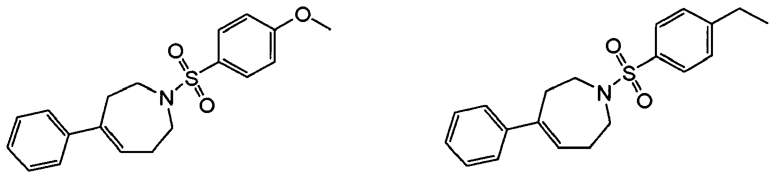 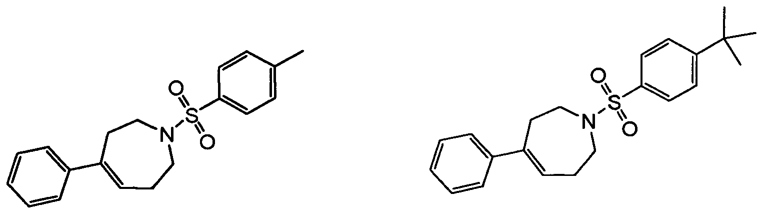 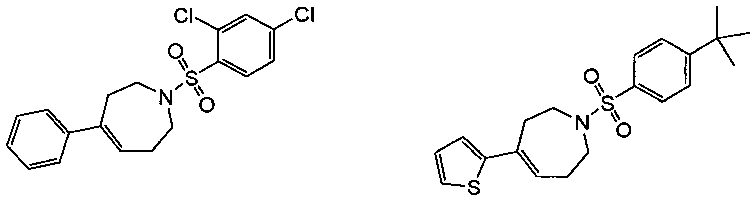 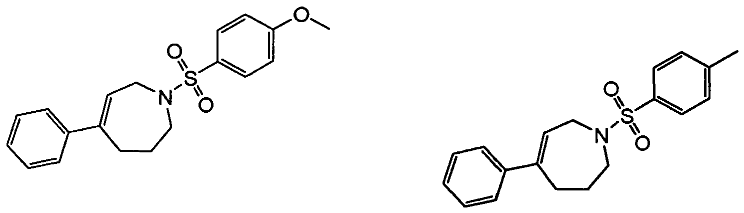 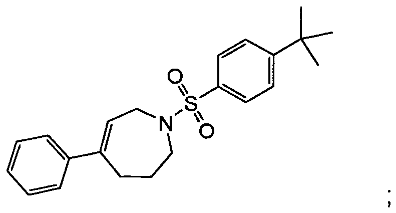 or a pharmaceutically acceptable salt or solvate thereof.

2. A pharmaceutical composition comprising at least one compound of claim 1, or a pharmaceutically acceptable salt or solvate thereof and at least one pharmaceutically acceptable carrier, adjuvant or vehicle.

3. The pharmaceutical composition of claim 2, further comprising one or more additional therapeutic agents.

4. The pharmaceutical composition of claim 3, wherein said additional therapeutic agents are one or more members selected from the group consisting of anti-obesity agents, antidiabetic agents, agents useful for treating metabolic syndrome, agents useful for treating a cardiovascular disease, cholesterol biosynthesis inhibitors, cholesterol absorption inhibitors, bile acid sequestrants, probucol derivatives, IBAT inhibitors, nicotinic acid receptor (NAR) agonists, ACAT inhibitors, cholesteryl ester transfer protein (CETP) inhibitors, low-denisity lipoprotein (LDL) activators, fish oil, water-soluble fibers, plant sterols, plant stanols and fatty acid esters of plant stanols.

5. A compound of claim 1 for use in therapy.

6. The compound of claim 5, for use in the treatment of metabolic syndromes, obesity, obesity-relatred disorders, hypertension, lipid disorders, type-II diabetes, insulin resistance and pancreatitis.

7. Use of a compound according of claim 1, for the manufacture of a medicament for the treatment of metabolic syndromes, obesity, obesity-relatred disorders, hypertension, lipid disorders, type-II diabetes, insulin resistance and pancreatitis.

## Patentansprüche

1. Eine Verbindung, ausgewählt aus der Gruppe, bestehend aus: 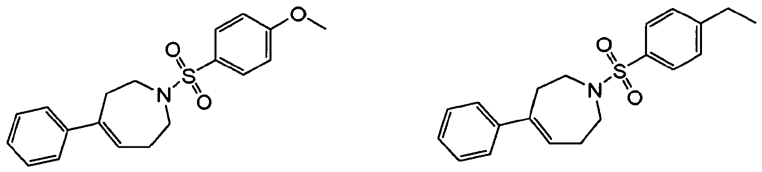 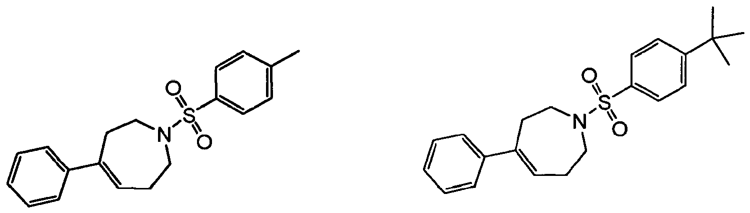 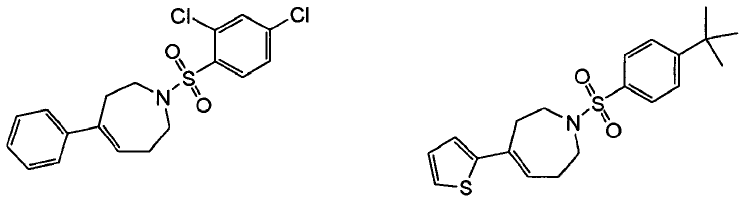 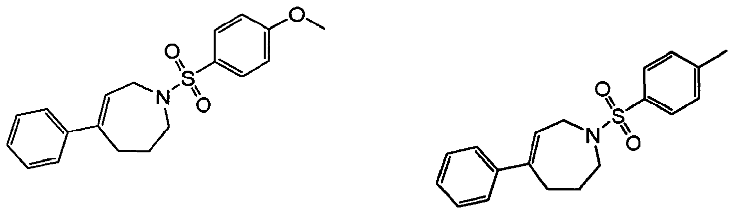 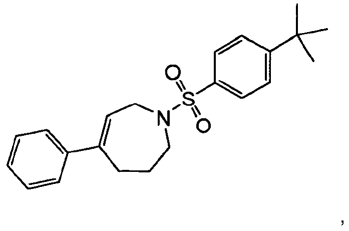 oder ein pharmazeutisch annehmbares Salz oder Solvat davon.

2. Eine pharmazeutische Zusammensetzung, umfassend wenigstens eine Verbindung nach Anspruch 1 oder ein pharmazeutisch annehmbares Salz oder Solvat davon und wenigstens einen pharmazeutisch annehmbaren Träger, einen pharmazeutisch annehmbaren Hilfsstoff oder ein pharmazeutisch annehmbares Vehikel.

3. Die pharmazeutische Zusammensetzung nach Anspruch 2, ferner umfassend ein oder mehrere zusätzliche therapeutische Mittel.

4. Die pharmazeutische Zusammensetzung nach Anspruch 3, wobei die zusätzlichen therapeutischen Mittel ein oder mehrere Elemente sind, ausgewählt aus der Gruppe, bestehend aus Mitteln gegen Adipositas, Antidiabetika, zur Behandlung von metabolischem Syndrom geeigneten Mitteln, zur Behandlung einer kardiovaskulären Erkrankung geeigneten Mitteln, Cholesterinbiosyntheseinhibitoren, Cholesterinabsorptionsinhibitoren, Gallensäuresequestriermitteln, Probucol-Derivaten, IBAT-Inhibitoren, Nikotinsäurerezeptor(NAR)-Agonisten, ACAT-Inhibitoren, Cholesterylestertransferprotein(CETP)-Inhibitoren, Low-Density-Lipoprotein(LDL)-Aktivatoren, Fischöl, wasserlöslichen Fasern, Pflanzensterolen, Pflanzenstanolen und Fettsäureestern von Pflanzenstanolen.

5. Eine Verbindung nach Anspruch 1 zur Verwendung in der Therapie.

6. Die Verbindung nach Anspruch 5 zur Verwendung bei der Behandlung von metabolischen Syndromen, Adipositas, mit Adipositas in Zusammenhang stehenden Störungen, Hypertonie, Lipidstörungen, Typ-II-Diabetes, Insulinresistenz und Pankreatitis.

7. Verwendung einer Verbindung gemäß Anspruch 1 zur Herstellung eines Medikaments zur Behandlung von metabolischen Syndromen, Adipositas, mit Adipositas in Zusammenhang stehenden Störungen, Hypertonie, Lipidstörungen, Typ-II-Diabetes, Insulinresistenz und Pankreatitis.

## Revendications

1. Composé sélectionné parmi le groupe consistant en: 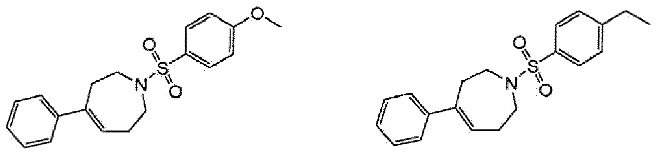 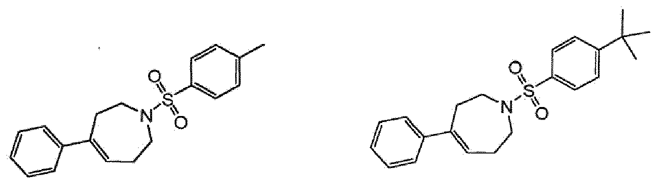 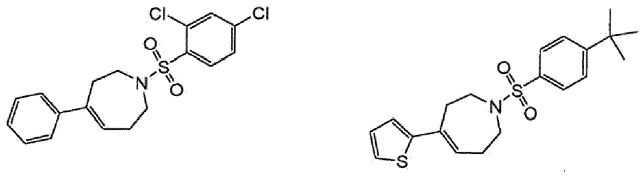 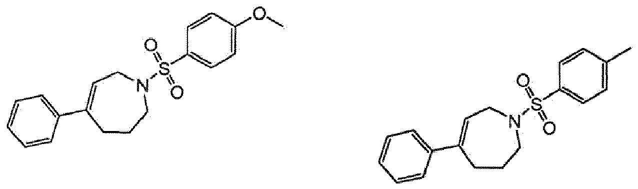 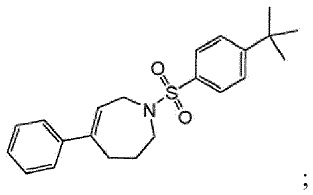 ou un sel ou un solvate pharmaceutiquement acceptable de celui-ci.

2. Composition pharmaceutique comprenant au moins un composé selon la revendication 1 ou un sel ou un solvate pharmaceutiquement acceptable de celui-ci et au moins un excipient, adjuvant ou véhicule pharmaceutiquement acceptable.

3. Composition pharmaceutique selon la revendication 2, comprenant en plus un ou plusieurs agents thérapeutiques supplémentaires.

4. Composition pharmaceutique selon la revendication 3, dans laquelle lesdits agents thérapeutiques supplémentaires sont un ou plusieurs membres sélectionnés parmi le groupe consistant en agents anti-obésité, agents antidiabétiques, agents utiles dans le traitement d'un syndrome métabolique, agents utiles dans le traitement d'une maladie cardiovasculaire, inhibiteurs de la biosynthèse du cholestérol, inhibiteurs d'absorption de cholestérol, séquestrants de l'acide biliaire, dérivés de probucol, inhibiteurs IBAT, agonistes des récepteurs de l'acide nicotinique (NAR), inhibiteurs d'ACAT, inhibiteurs de la protéine de transfert des esters de cholestérol (CETP), activateurs des lipoprotéines basse densité (LDL), huile de poisson, fibres solubles dans l'eau, stérols végétaux, stanols végétaux et esters d'acides gras de stanols végétaux.

5. Composé selon la revendication 1, à utiliser en thérapie.

6. Composé selon la revendication 5, à utiliser dans le traitement de syndromes métaboliques, de l'obésité, de troubles associés à l'obésité, de l'hypertension, de troubles lipidiques, du diabète de type II, de l'insulino-résistance et de la pancréatite.

7. Utilisation d'un composé selon la revendication 1, dans la fabrication d'un médicament pour le traitement de syndromes métaboliques, de l'obésité, de troubles associés à l'obésité, de l'hypertension, de troubles lipidiques, du diabète de type II, de l'insulino-résistance et de la pancréatite.
